Europäisches Patentamt

European Patent Office

Office européen des brevets

(11) Publication number: **0 255 405**
**A1**

(12) **EUROPEAN PATENT APPLICATION**

(21) Application number: **87306839.9**

(22) Date of filing: **31.07.87**

(51) Int. Cl.⁴: **C 12 P 19/04**
**C 08 B 37/00**

(30) Priority: **01.08.86 US 891914**

(43) Date of publication of application:
**03.02.88 Bulletin 88/05**

(84) Designated Contracting States:
**BE CH DE FR GB IT LI NL**

(71) Applicant: **CELGENE CORPORATION**
**7 Powderhorn Drive**
**Warren New Jersey (US)**

(72) Inventor: **Voloch, Marcio**
**44 Carriage Place**
**Edison New Jersey (US)**

**Stirling, David I.**
**4 Lois Place**
**Fanwood New Jersey (US)**

**Pegram, Catherine**
**114 Maple Avenue**
**Newark New Jersey (US)**

(74) Representative: **De Minvielle-Devaux, Ian Benedict Peter et al**
**CARPMAELS & RANSFORD 43, Bloomsbury Square**
**London WC1A 2RA (GB)**

The microorganism(s) has (have) been deposited with American Type Culture Collection under number(s) ATCC-53412, AT-39893.

(54) **Production of heat-modified heteropolysaccharides.**

(57) This invention provides a process which involves cultivating a strain of Methylophilus viscogenes under growth conditions to produce an aqueous solution of an accumulated quantity of an exopolysaccharide, such as heteropolysaccharide Poly 54, and heating the aqueous solution to produce heat-modified exopolysaccharide which imparts enhanced pseudoplastic and thixotropic properties to aqueous solutions.

EP 0 255 405 A1

Bundesdruckerei Berlin

**Description**

## PRODUCTION OF HEAT-MODIFIED HETEROPOLYSACCHARIDES

### BACKGROUND OF THE INVENTION

Methylophilus methylotrophus is an obligate methylotroph which uses the ribulose monophosphate pathway(RMP) cycle of formaldehyde fixation. Methylophilus methylotrophus Strain AS-I is a gram negative, nonpigmented rod with a single polar flagellum.

Although methanol and other $C_1$-compounds generally are viewed as substrates for the production of single cell protein, the factors that qualify $C_1$-compounds for SCP manufacture also recommend them as a potential feedstock for the production of accumulated extracellular metabolites such as exopolysaccharides. A number of microbial processes for the Conversion of methanol to value-added products have been described but generally these are low-volume/high-priced compounds such as aminoacids. Thus, J. Bolbot and C. Anthony in Proc. Soc. Gen. Microbial, 5, 43(1978) found that a pyruvate dehydrogenase lacking mutant of Pseudomonas AMI could accumulate the aminoacids alanine and valine during growth on methanol. Y. Tani et al in Agric. Giol. Chem., 42, 2275 (1978) have described the production of up to 5.2 grams per liter of L-serine from methanol employing an Arthrobacter globiformis strain. Up to the present time there has not been any report of $C_1$ compound bioconversion to a commodity type of bulk chemical.

Accordingly, it is an object of this invention to provide a fermentation process for the bioconversion of a growth carbon source to an accumulated quantity of an exopolysaccharide metabolite.

It is another object of this invention to provide a process for the bio-oxidation of a $C_1$-compound to an accumulated quantity of an exopolysaccharide, and for the heat-modification of the exopolysaccharide to form a novel heteropolysaccharide.

It is a further object of this invention to provide a novel form of heteropolysaccharide Poly 54 which imparts enhanced pseudoplastic and thixotropic properties to aqueous solutions.

Other objects and advantages of the present invention shall become apparent from the accompanying description and examples.

### DESCRIPTION OF THE INVENTION

One or more objects of the present invention are accomplished by the provision of a process for increasing the viscosity of an aqueous solution containing a heteropolysaccharide produced by aerobic cultivation of a Methylophilus viscogenes bacterial strain, which comprises heating the aqueous solution at a temperature between about 60°-I50°C for a time period sufficient to achieve an increase in solution viscosity.

The aqueous solution can contain up to about 50 weight percent of a water-miscible solvent, such as methanol, acetone, tetrahydrofuran, dimethylformamide, and the like.

In another embodiment, this invention provides a process which comprises aerobically cultivating Methylophilus viscogenes strain ATCC 39893 or a variant thereof in an aqueous nutrient medium containing a growth carbon source (e.g., a $C_1$-compound or fructose) to produce an aqueous solution of extracellular heteropolysaccharide Poly 54, and heating the aqueous solution at a temperature between about 60°-I50°C to produce an aqueous solution of heat-modified heteropolysaccharide Poly 54.

The time period for heat-treatment of a heteropolysaccharide in a present invention process embodiment will vary in the range between about 0.I-I0 hours.

Heat-modified heteropolysaccharide Poly 54 is a novel composition of matter.

As an optional procedure, the whole cells can be removed from the culture medium prior to the heat-treatment stage. The whole cells can be removed from the culture medium by conventional means such as centrifugation or ultrafiltration, and then the cell-free aqueous solution of exopolysaccharide metabolite can be subjected to the subsequent processing procedures for heat-treatment and product recovery.

The term "$C_1$-compound" as employed herein refers to an organic compound which does not contain any carbon-carbon bonds, such as methanol, formaldehyde, formate, formamide, carbon monoxide, dimethyl ether, methylamine, dimethylamine, trimethylamine and trimethylamine N-oxide.

The term "exopolysaccharide" as employed herein refers to a polysaccharide which accumulates as an extracellular metabolite in a fermentation medium, as exemplified by xanthan gum.

The term "heteropolysaccharide" as employed herein refers to a polysaccharide which is composed of at least two different kinds of monosaccharidic units, such as mannose and galactose.

The term "heat-modified" as employed herein refers to the physicochemical changes introduced in a polysaccharide by heat-treatment in an aqueous medium, as compared to the same polysaccharide before heat-treatment.

In another embodiment, this invention provides a process which comprises heating an aqueous slurry of heteropolysaccharide Poly 54 at a temperature between about 60°-I50°C to produce heat-modified heteropolysaccharide Poly 54 in solid form. The aqueous phase of the slurry can contain a water-miscible organic solvent to minimize the content of dissolved heteropolysaccharide Poly 54 during the heat-treatment phase.

A present invention heat-modified heteropolysaccharide can be recovered from an aqueous solution by any convenient procedure such as freeze-drying, or precipitation with a water-soluble organic solvent, e.g.,

acetone, methanol, ethanol, and the like. Precipitation of a heat-modified heteropolysaccharide can also be effected by treatment of the solution with a salt of a polyvalent metal such as calcium or barium.

A crude heat-modified heteropolysaccharide can be redissolved in water, and the aqueous solution then subjected to dialysis and lyophilization to provide a purified product.

Heat-modified heteropolysaccharide Poly 54 product has an average molecular weight in the range between about 800,000-1,500,000.

Heat-modified heteropolysaccharide Poly 54 exhibits a 0.25 weight percent viscosity of at least about 8000 cps at a shear rate of 0.1 reciprocal second, as measured in a deionized aqueous medium with Brookfield RVT Viscometer Spindle No. 31 at 25°C (with small sample adapter). An aqueous solution of heat-modified heteropolysaccharide Poly 54 is colorless, transparent and exhibits pseudoplastic and thixotropic viscosity properties. The aqueous solution viscosity properties are stable at temperatures up to about 130°C, and at pH values up to about 12.

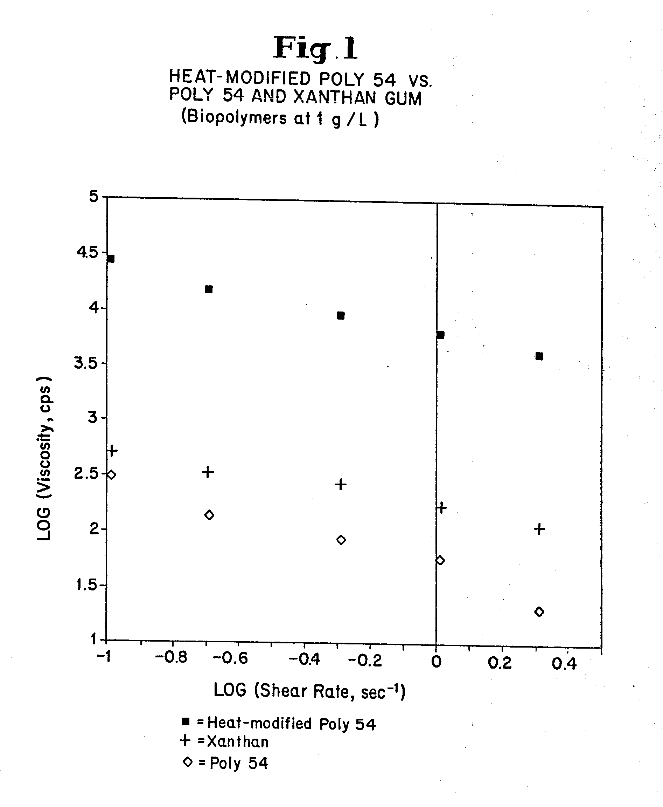

Under low shear rate conditions, heat-modified heteropolysaccharide Poly 54 exhibits an apparent viscosity which typically is about 5-50 times that of commercial xanthan gum. The comparative viscosity properties of heteropolysaccharide Poly 54, heat-modified heteropolysaccharide Poly 54 and commercial xanthan gum (e.g., Kelzan) under shear conditions are illustrated in Figure 1.

Other physicochemical and structural features of heat-modified heteropolysaccharide Poly 54 are as follows:

A. Constituent Sugars(molar ratio)
glucose      10
galactose    7-10
mannose      1-3
glycuronic acid      1-3

Heat-modified heteropolysaccharide Poly 54 contains between about 1-3 weight percent of nitrogen. Heat-modified heteropolysaccharide Poly 54 has no pyruvate or protein content, and it contains about one acetyl group per four monosaccharide units. The glycuronic acid constituent comprises glucuronic acid and/or galacturonic acid and/or mannuronic acid.

B. Elemental Analysis(%)[1]
C    39.2
H    6.1
O    43.1
N    2.17

C. Melting Point
No clear melting point, and decomposes above a temperature of about 150°C.

D. Infrared Spectrum
Band at 1740 cm$^{-1}$ which may indicate aliphatic ester groups.
Bands at 1650 cm$^{-1}$ and 1540 cm$^{-1}$ which may indicate amide groups.

E. Ultraviolet Absorption Spectrum
No detectable peaks in ultraviolet range of wavelengths.

F. Solubility Properties
Soluble in water, but insoluble in all common organic solvents.

G. Specific Rotation
No measurable specific rotation.

Deacetylated Heteropolysaccharides
In another embodiment, this invention provides a process which comprises aerobically cultivating a Methylophilus viscogenes bacterial strain in an aqueous nutrient medium containing a growth carbon source to produce an aqueous solution of an accumulated quantity of extracellular heteropolysaccharide, adjusting the pH of the aqueous solution into the alkaline range to deacetylate the heteropolysaccharide, and heating the aqueous solution at a temperature between about 60°-150°C to produce an aqueous solution of heat-modified deacetylated heteropolysaccharide product.

When the bacterial strain is Methylophilus viscogenes ATCC 39893 or a variant thereof, and the growth carbon source is methanol, the resultant product corresponds to heat-modified deacetylated heteropolysaccharide Poly 54.

In another embodiment, this invention provides a process which comprises heating an aqueous solution of deacetylated heteropolysaccharide Poly 54 at a temperature between about 60°-150°C to produce heat-modified deacetylated heteropolysaccharide Poly 54.

[1] Average values; ash corrected.

In a further embodiment, this invention provides a process which comprises heating an aqueous slurry of deacetylated heteropolysaccharide Poly 54 at a temperature between about 60°-l50°C to produce heat-modified deacetylated heteropolysaccharide Poly 54 in solid form.

The biosynthesis of exopolysaccharides by Methylophilus viscogenes bacterial strains, and the chemical conversion of the exopolysaccharides to deacetylated heteropolysaccharides, is described in copending patent application Serial Number 835,8l3, filed March 3, l986; incorporated by reference.

The alkaline treatment procedure to produce the deacetylated exopolysaccharide compound can be accomplished by adjusting the pH of an aqueous solution of the exopolysaccharide intermediate to a pH value between about 7-l3, preferably to a pH between about 8-l2 with an alkaline reagent at a temperature between about 20°-l00°C for a treatment period of sufficient duration to effect hydrolytic deacetylation of the exopolysaccharide. A typical reaction time is between about 0.l-2 hours. The alkaline reagent can be ammonia or an organic amine, or a basic inorganic compound such as sodium hydroxide, potassium carbonate, and the like.

The heat-treatment period to produce a heat-modified deacetylated heteropolysaccharide will vary in the range between about 0.l-l0 hours, and typically will have a duration between about 0.5-5 hours.

A present invention composition such as heat-modified deacetylated heteropolysaccharide Poly 54 can be recovered as a solid product from an aqueous medium by conventional means, such as by dilution of the aqueous medium with a water-miscible organic solvent to precipitate the product. Illustrative of suitable organic solvent diluents are methanol, ethanol, propanol, acetone, tetrahydrofuran, and the like.

The heat-modified deacetylated heteropolysaccharide also can be precipitated by treatment of the aqueous solution with a metal salt such as calcium carbonate or barium hydroxide.

The heat-modified deacetylated heteropolysaccharide product contains carboxylic acid groups, and can exist either as a metal salt or in the free acid form.

Heat-modified deacetylated heteropolysaccharide Poly 54 of the present invention is a novel composition of matter.

Heat-modified deacetylated heteropolysaccharide Poly 54 has an average molecular weight in the range between about 800,000-l,500,000.

Heat-modified deacetylated heteropolysaccharide Poly 54 exhibits a 0.25% weight percent viscosity of at least about 8000 cps at a shear rate of 0.l reciprocal second, as measured in a deionized aqueous medium with Brookfield Spindle No. 3l at 25°C (with small sample adapter). An aqueous solution of heat-modified deacetylated heteropolysaccharide Poly 54 is colorless, transparent and exhibits pseudoplastic and thixotropic viscosity properties. The aqueous solution viscosity properties are stable at temperatures up to l30°C, and at pH values up to about l2.

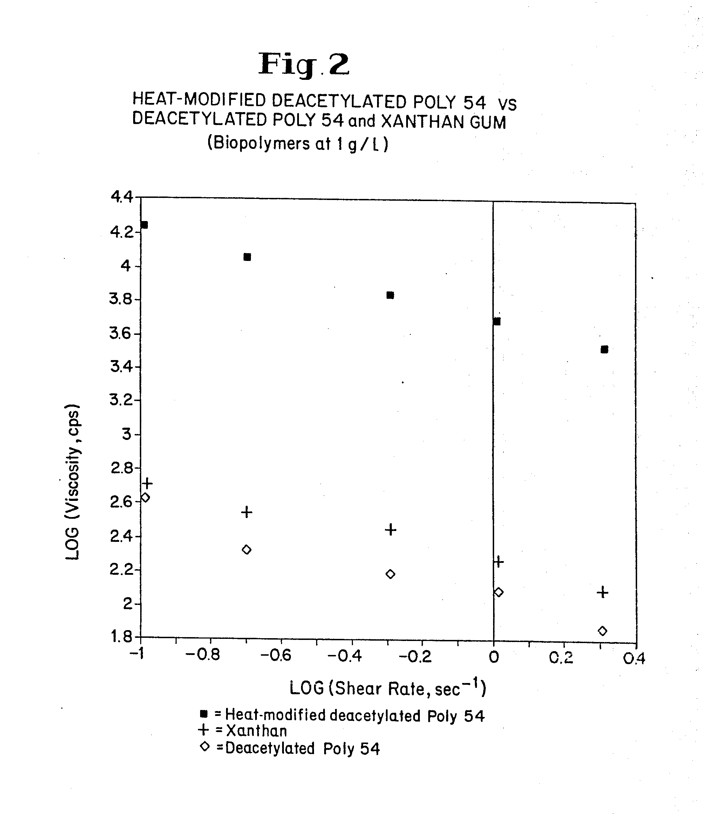

Under low shear rate conditions, heat-modified deacetylated heteropolysaccharide Poly 54 exhibits an apparent viscosity which typically is about 5-50 times that of commercial xanthan gum. The comparative viscosity properties of deacetylated heteropolysaccharide Poly 54, heat-modified deacetylated heteropolysaccharide Poly 54 and commercial xanthan gum (e.g., Kelzan) under shear conditions are illustrated in Figure 2.

Other physicochemical and structural features of heat-modified deacetylated heteropolysaccharide Poly 54 are as follows:

A. Constituent Sugars(mole%)

| glucose | l0 |
| galactose | 7-l0 |
| mannose | l-3 |
| glycuronic acid | l-3 |

The glycuronic acid constituent comprises glucuronic acid and/or galacturonic acid and/or mannuronic acid.

Heat-modified deacetylated heteropolysaccharide Poly 54 contains no pyruvate or protein, and it contains less than about one acetyl group per four monosaccharide units. Usually the alkaline treatment removes all of the acetyl groups present in the original heteropolysaccharide Poly 54. Partially deacetylated Poly 54 also can be produced and heat-treated.

B. Elemental Analysis(%)[2]

| C | 38.22 |
| H | 5.57 |
| O | 40.4l |
| N | l.58 |

C. Melting Point

No clear melting point, and decomposes above a temperature of about l50°C.

D. Infrared Spectrum

Band at l740 cm$^{-1}$ which may indicate aliphatic ester groups.
Bands at l650 cm$^{-1}$ and l540 cm$^{-1}$ which may indicate amide groups.

[2] Ash corrected.

E. Ultraviolet Absorption Spectrum
 Broad $\lambda_{max}$ peak at 254 mu.


F. Solubility Properties
 Soluble in water, but insoluble in all common organic solvents.


G. Specific Rotation
 No measurable specific rotation.


The Nature of the Heat-treatment Modification

 The heat-treatment of a heteropolysaccharide Poly 54 type of exopolysaccharide does not appear to change the chemical constituency of the heteropolysaccharide molecules. Elemental analyses are the same for the heteropolysaccharide before and after heat-treatment.

 There is no significant differences in ultraviolet and infrared spectra for the heteropolysaccharide before and after heat-treatment.

 The differences that are apparent for the heteropolysaccharide before and after heat-treatment appear to relate to the physicochemical conformation of the heteropolysaccharide molecules (eg., random coil vs. rod shape). Another parameter is the presence of intramolecular and intermolecular electrostatic bondings which have mechanical and functional properties different than non-specific entanglements.

 It is believed that the heat-treatment of an exopolysaccharide such as heteropolysaccharide Poly 54 in an aqueous medium changes the electrostatic bonding within a molecule and between molecules. It is presumed that the molecules physically rearrange and assume a thermodynamically more favorable geometric conformation, one which is permanent as indicated by physical measurements and NMR spectra.

 A heat-modified heteropolysaccharide is a more effective viscosity-enhancing agent than the corresponding unmodified heteropolysaccharide.

 An aqueous solution of a heat-modified heteropolysaccharide exhibits a greater tolerance to salt than the corresponding unmodified heteropolysaccharide.

 The present invention contemplates other novel processing embodiments for the production of heat-modified heteropolysaccharides which exhibit improved viscosity-enhancing properties in aqueous media.

 In one embodiment, this invention provides a process which comprises (I) aerobically cultivating a Methylophilus viscogenes bacterial strain in an aqueous nutrient medium containing a growth carbon source to produce a culture medium aqueous solution of an accumulated quantity of extracellular heteropolysaccharide, (2) removing water from the culture medium aqueous solution to provide a concentrated aqueous solution containing between about 5-20 grams per liter of extracellular heteropolysaccharide, and (3) heating the concentrated aqueous solution at a temperature between about 60°-I50°C to produce an aqueous solution of heat-modified heteropolysaccharide product.

 The steps (2) and (3) functions can be conducted as a combined procedure. The removal of water in step(2) can be accomplished by techniques such as vacuum distillation or reverse osmosis.

 In another embodiment, this invention provides a process which comprises (I) aerobically cultivating a Methylophilus viscogenes bacterial strain in an aqueous nutrient medium containing a growth carbon source to produce a culture medium aqueous solution of an accumulated quantity of extracellular heteropolysaccharide, (2) adjusting the pH of the aqueous solution into the alkaline range to deacetylate the heteropolysaccharide, (3) removing water from the culture medium aqueous solution to provide a concentrated aqueous solution containing between about 5-20 grams per liter of extracellular deacetylated heteropolysaccharide, and (4) heating the concentrated aqueous solution at a temperature between about 60°-I50°C to produce an aqueous solution of heat-modified deacetylated heteropolysaccharide product.

 The steps (3) and (4) functions can be conducted as a combined procedure, e.g., vacuum distillation with heating.

 If Methylophilus viscogenes ATCC 39893 or a variant thereof is utilized as the bacterial strain in the above described two process embodiments, then the products are concentrated aqueous solutions of heat-modified heteropolysaccharide Poly 54 and heat-modified deacetylated heteropolysaccharide Poly 54, respectively.

 As illustrated in Figures I-2, unmodified heteropolysaccharide Poly 54 and deacetylated Poly 54 have apparent viscosities which are similar to that of xanthan gum at concentrations below 0.4% (w/v). At low concentrations (e.g., less than 0.25%, w/v) xanthan gum solutions can exhibit somewhat greater apparent viscosities. As polymer concentration increases (e.g., greater than 0.5 w/v) the apparent viscosity of heteropolysaccharide Poly 54 solutions is significantly greater than that of equivalent xanthan gum solutions, e.g., an apparent viscosity which is 5-30 times that of xanthan gum.


Species Methylophilus Viscogenes

 A detailed description of the novel species Methylophilus viscogenes and its production of exopolysaccharides is elaborated in copending patent application Serial Number 826,530, filed February 6, I986, incorporated by reference.

 Strains of Methylophilus viscogenes exhibit a novel combination of properties that distinguish them from other methylotrophic bacteria. A <u>Methylophilus viscogenes</u> bacterium such as strain ATCC 39893 is a type I facultative methylotroph which utilizes the ribulose monophosphate pathway of $C_1$ assimilation, and which typically has a growth rate doubling time of I-3 hours at 35°-40°C.

Strain ATCC 39893 grows on methanol, fructose and glucose. In addition, it will grow on a wider variety of heterotrophic substrates (e.g., succinate and pyruvate) when an exogenous energy supply in the form of formate or methanol is present. This is a property not previously described for any known microorganism. For purposes of identification, bacteria manifesting this phenomenon are herein termed "latent facultative methylotrophs".

Bacteria having the characteristics comprising those of strain ATCC 39893 and variants thereof are further identified by non-slimy growth of pale orange colonies on solid media. Another identifying characteristic of a strain ATCC 39893 type of methylotrophic bacterium is a hexulose phosphate synthase/hexulose phosphate isomerase activity (J.P. Van Dijken et al; FEMS. Microbiol. Lett., 4, 97, 1978) of at least about 400 nanomoles of NADH formed per minute per milligram of protein.

Another significant property of a strain ATCC 39893 type of facultative methylotroph is the ability to bioconvert a methanol substrate into an accumulated quantity of an exopolysaccharide which imparts pseudoplastic and thixotropic properties to aqueous solutions. A strain ATCC 39893 bacterium is particularly unique in its ability to produce a large quantity of accumulated exopolysaccharide in the cultivation medium, in the presence or absence of cell growth and under a variety of culture conditions. A strain ATCC 39893 type of bacterium has the inherent ability to divert a large percentage of available carbon source to biosynthesis of an exopolysaccharide.

The term "methylotroph" as employed herein refers to a microorganism which is capable of growing non-autotrophically on carbon compounds having one or more carbon atoms but no carbon-carbon bonds. "Autotrophic" refers to growth on a carbon dioxide substrate.

The term "facultative methylotroph" as employed herein refers to a methylotroph which is capable of growth on one or more heterotrophic substrates, e.g., fructose.

The term "variant" as employed herein refers to a <u>Methylotrophus viscogenes</u> strain which is phenotypically and/or genotypically different from the parent strain from which it is derived, and which exhibits the methylotrophic parent strain ability to biosynthesize an exopolysaccharide.

Illustrative of a variant strain is ATCC 534l2, which is a mutant of strain ATCC 39893.

During routine subculturing of a rifampicin-resistant strain of ATCC 39893, a spontaneous variant is formed on the growth medium. The variant is detected due to its unusual colony morphology on methanol/ammonium mineral salts agar plates.

Colonies of ATCC 39893 grown on such plates are small, round, pale orange and non-slimy in nature. Colonies of the variant strain are larger (about 4 mm in diameter). off-white to clear in color, and very slimy in nature.

The new strain (ATCC 534l2) is determined to be a variant of ATCC 39893 by exhibiting rifampicin-resistance, equivalent cultural and biochemical characteristics, and finally by chromosomal DNA:DNA hybridization with ATCC 39893.

Variant strain ATCC 534l2 is capable of constitutively synthesizing an exopolysaccharide equivalent to heteropolysaccharide Poly 54. Strain ATCC 534l2 has an optimal growth rate which is 2-3 times that of ATCC 39893.

Subcultures of accession Number ATCC 39893 and ATCC 534l2 strains can be obtained upon request from the permanent microorganism collection of the American Type Culture Collection, l230l Parklawn Drive, Rockville, Maryland 20852. The culture depositions are in accordance with the requirements of the Budapest Treaty for the purposes of patent procedure.

The following examples are further illustrative of the present invention. The components and specific ingredients are presented as being typical, and various modifications can be derived in view of the foregoing disclosure within the scope of the invention.

For growth of methanol-assimilating bacteria a mineral salts medium(MS) (Table I) is employed. The medium is either supplemented with I g/L potassium nitrate, giving nitrate mineral salts medium(NMS), or I g/L ammonium chloride, giving ammonium mineral salts medium(AMS). The carbon source, methanol, preferably is present at a concentration between 0.2-0.5% (v/v).

For solid media, l7 g/L of Difco bacto-agar is added to the basic mineral salts medium (minus phosphates) prior to sterilization. Sterile phosphate solution is added aseptically to the sterile mineral salts medium on cooling.

Fermentation procedures are conducted in a B. Braun instruments biostat S fermentor (6 liters). A 3 liter working volume is used, and methanol is added aseptically after sterilization of the fermentor. A stirring rate of 500 rpm is routinely used with an air delivery rate of 460 mls m−1. The fermentor is equipped with pH and dissolved oxygen control Hexulose phosphate synthase/hexulose phosphate isomerase are assayed simultaneously, since there is no assay for hexulose 6-phosphate which is the product of hexulose phosphate synthase activity. Hexulose phosphate isomerase converts hexulose 6-phosphate to glucose 6-phosphate which can be estimated using glucose 6-phosphate dehydrogenase and following the concomitant NADP+ reduction at 340 nm. The enzyme assay solution contains (final conc.): phosphate buffer 50 mM, pH 7.2; magnesium chloride 2.5 mM; glucose 6-phosphate dehydrogenase 0.7 units; phosphoglucoisomerase 0.75 units; phosphoribose isomerase l.75 units; ribose 5-phosphate 5 mM; NADP+ 0.25 mM; and formaldehyde

6

5mM.

Any presence of hydroxypyruvate reductase is indicative of the serine pathway of formaldehyde assimilation in the microorganism. This is determined by an enzyme assay solution which contains (final conc.): potassium phosphate, IM, pH 6.3; lithium hydroxy pyruvate 0.0l M; and NADH 2mM. NADH disappearance is measured at 340 nm.

The estimation of glycerol and dihydroxyacetone is accomplished with glycerol dehydrogenase/dihydroxyacetone reductase (glycerol:NAD+ 2-oxidoreductase, EC l.l.l.6). The enzyme is derivared from Enterobacter aerogenes, and is available from Sigma Biochemicals.

For the estimation of glycerol, the reaction mixture (l.0 ml) contains 50 mole TRIS-HCl buffer (Sigma Biochemicals), pH 9.7; 0.2 mole NAD+; l unit enzyme; and l5 mole glycerol (or test solution/fermentation broth, 20-50 l). Assays are initiated by addition of substrate and followed by monitoring increasing absorbance at 340 nm in a Beckman Model 25 UV spectrophotometer.

For the estimation of dihydroxyacetone, the reaction mixture (0.l ml) contains 50 mole phosphate buffer, pH 6.0; 0.5 mole NADH; l unit enzyme; l5 mole dihydroxyacetone (or test solution/fermentation broth, 20-50 ml). Assays are initiated by addition of substrate and followed by monitoring decreasing absorbance at 340 nm.

Rheological measurements on aqueous solutions of polysaccharides are made on a Wells/Brookfield cone/plate digital viscometer or a Rheomat 30 viscometer fitted with a coaxial cylinder sensor system.

The xanthan gum employed for comparative viscosity measurements is Kelzan, which is a commercial product sold by Kelco Co., San Diego, California.

Analysis of the monosaccharide content of an exopolysaccharide is accomplished by a hydrolysis-gas liquid chromatography method.

## EXAMPLE I

This Example illustrates the culturing of a Methylotrophus viscogenes strain to form an accumulated quantity of exopolysaccharide metabolite, and the subsequent heat-treatment to form heat-modified exopolysaccharide.

A 500 ml inoculum of strain ATCC 39893 is grown employing MS medium, one gram per liter of ammonium chloride, and 0.5% (v/v) methanol as a growth carbon source. The flask is incubated at 37°C for two days. A l4 liter New Brunswick Microferm fermentor containing l0 liters of MS medium, one gram per liter of ammonium chloride, and 0.5% (v/v) methanol is inoculated with the above culture.

Initial fermentation conditions are as follows: 37°C, agitation 200 rpm, air flow 2 liters/minute, and pH 7.0. The pH is controlled at about 7.0 during the fermentation. Methanol is added intermittently to provide a concentration of 0.5% (v/v) whenever the fermentation medium becomes carbon exhausted. The concentration of methanol is determined by gas-liquid chromatography. After 24-36 hours of fermentation, the agitation rate is increased to 400 rpm.

The fermentation is terminated when methanol is no longer being utilized by the culture (usually 5-7 days).

The aqueous solution is transferred to an autoclave and heated at l2l°C for a period of one hour.

After the heat-treatment period, the aqueous solution is diluted with aqueous isopropanol (2:l alcohol:water) to precipitate the heteropolysaccharide product. The precipitate is collected by filtration. The precipitate is washed with about one volume of isopropanol, then air dried or dried at 55°C in a forced air oven. The dried solids subsequently are milled to a powder in a Wiley Mill or hammer mill. The physicochemical properties of the heat-treated heteropolysaccharide product correspond to those described hereinabove for heat-modified heteropolysaccharide Poly 54.

## EXAMPLE II

This Example illustrates the culturing of a Methylotrophus viscogenes strain to form an accumulated quantity of exopolysaccharide metabolite, and the subsequent alkaline treatment and heat-treatment to form heat-modified deacetylated heteropolysaccharide.

A 500 ml inoculum of strain ATCC 39893 is grown employing MS medium, one gram per liter of ammonium chloride, and 0.5% (v/v) methanol as a growth carbon source. The flask is incubated at 37°C for two days. A l4 liter New Brunswick Microferm fermentor containing l0 liters of MS medium, one gram per liter of ammonium chloride, and 0.5% (v/v) methanol is inoculated with the above culture.

Initial fermentation conditions are as follows: 37°C, agitation 200 rpm, air flow 2 liters/minute, and pH 7.0. The pH is controlled at about 7.0 during the fermentation. Methanol is added intermittently to provide a concentration of 0.5% (v/v) whenever the fermentation medium becomes carbon exhausted. The concentration of methanol is determined by gas-liquid chromatography. After 24-36 hours of fermentation, the agitation rate is increased to 400 rpm.

The fermentation is terminated when methanol is no longer being utilized by the culture (usually 5-7 days). The pH of the aqueous fermentation medium is adjusted to pH l2 with sodium hydroxide, and after two hours at pH l2 the fermentation medium is neutralized with hydrochloric acid to provide an aqueous solution of diacetylated heteropolysaccharide Poly 54.

The aqueous solution is transferred to an autoclave and heated at l2l°C for a period of one hour.

After the heat-treatment period, the aqueous solution is diluted with aqueous isopropanol (2:l alcohol:water) to precipitate the heteropolysaccharide product. The precipitate is collected by filtration. The precipitate is washed with about one volume of isopropanol, then air dried or dried at 55°C in a forced air oven.

The dried solids subsequently are milled to a powder in a Wiley Mill or hammer mill. The physicochemical properties of the heat-treated deacetylated heteropolysaccharide product correspond to those described hereinabove for heat-modified deacetylated heteropolysaccharide Poly 54.

## TABLE I

### MS MEDIUM

| | |
|---|---|
| $MgSO_4 \cdot 7H_2O$ | 1 g |
| $CaCl_2$ | 0.2 g |
| $Na_2HPO_4$ | 0.33 g |
| $KH_2PO_4$ | 0.26 g |
| FeEDTA | 5.0 mg |
| $CuCl_2 \cdot H_2O$ | 2.0 mg |
| $Na_2MoO_4 \cdot 2H_2O$ | 2.0 mg |
| $FeSO_4 \cdot 7H_2O$ | 500 g |
| $ZnSO_4 \cdot 7H_2O$ | 400 g |
| $MnCl_2 \cdot 4H_2O$ | 20 g |
| $H_3BO_4$ | 15 g |
| $CoCl_2 \cdot 6H_2O$ | 50 g |
| $NiCl_2 \cdot 6H_2O$ | 10 g |
| EDTA | 250 g |
| $H_2O$ | 1 liter pH 6.8 |

## Claims

1. A process for increasing the viscosity of an aqueous solution containing a heteropolysaccharide produced by aerobic cultivation of a Methylophilus viscogenes bacterial strain, which comprises heating the aqueous solution at a temperature between about 60°-150°C for a time period sufficient to achieve an increase in solution viscosity.

2. A process in accordance with claim I wherein the bacterial strain is Methylophilus viscogenes ATCC 39893 or ATCC 53412 or a variant thereof.

3. A process in accordance with claim I wherein the aqueous solution contains up to about 50 weight percent of water-miscible organic solvent.

4. A process which comprises aerobically cultivating Methylophilus viscogenes strain ATCC 39893 or a variant thereof in an aqueous nutrient medium containing methanol as a growth carbon source to produce an aqueous solution of extracellular heteropolysaccharide Poly 54, and heating the aqueous solution at a temperature between about 60°-150°C to produce an aqueous solution of heat-modified heteropolysaccharide Poly 54.

5. A process which comprises heating an aqueous slurry of heteropolysaccharide Poly 54 at a temperature between about 60°-l50°C to produce heat-modified heteropolysaccharide Poly 54 in solid form.

6. Heat-modified heteropolysaccharide Poly 54.

7. Heat-modified heteropolysaccharide Poly 54 which exhibits a 0.25% weight percent viscosity of at least about 8000 cps at a shear rate of 0.l reciprocal second, as measured in a deionized aqueous medium with Brookfield Spindle No. 3l at 25°C.

8. Heat-modified heteropolysaccharide Poly 54 which exhibits an aqueous solution viscosity in centipoises that is at least twice the viscosity measured for an equivalent aqueous solution of heteropolysaccharide Poly 54 over a concentration range of 0.005-5 weight percent and at a shear rate between about 0.0l-l000 reciprocal seconds.

9. A process which comprises aerobically cultivating a Methylophilus viscogenes bacterial strain in an aqueous nutrient medium containing a growth carbon source to produce an aqueous solution of an accumulated quantity of extracellular heteropolysaccharide, adjusting the pH of the aqueous solution into the alkaline range to deacetylate the heteropolysaccharide, and heating the aqueous solution at a temperature between about 60°-l50°C to produce an aqueous solution of heat-modified deacetylated heteropolysaccharide product.

l0. A process in accordance with claim 9 wherein the growth carbon source is methanol.

ll. A process in accordance with claim 9 wherein the bacterial strain is Methylophilus viscogenes ATCC 39893 or a variant thereof, and the product is heat-modified deacetylated heteropolysaccharide Poly 54.

l2. A process which comprises heating an aqueous solution of deacetylated heteropolysaccharide Poly 54 at a temperature between about 60°-l50°C to produce heat-modified deacetylated heteropolysaccharide Poly 54.

l3. A process which comprises heating an aqueous slurry of deacetylated heteropolysaccharide Poly 54 at a temperature between about 60°-l50°C to produce heat-modified deacetylated heteropolysaccharide Poly 54 in solid form.

l4. Heat-modified deacetylated heteropolysaccharide Poly 54.

l5. Heat-modified deacetylated heteropolysaccharide Poly 54 which exhibits a 0.25% weight percent viscosity of at least about 8000 cps at a shear rate of 0.l reciprocal second, as measured in a deionized aqueous medium with Brookfield Spindle No. 3l at 25°C.

l6. Heat-modified deacetylated heteropolysaccharide Poly 54 which exhibits an aqueous solution viscosity in centipoises that is at least twice the viscosity measured for an equivalent aqueous solution of deacetylated heteropolysaccharide Poly 54 over a concentration range of 0.005-5 weight percent and at a shear rate between about 0.0l-l000 reciprocal seconds.

l7. A process which comprises (l) aerobically cultivating a Methylophilus viscogenes bacterial strain in an aqueous nutrient medium containing a growth carbon source to produce a culture medium aqueous solution of an accumulated quantity of extracellular heteropolysaccharide, (2) removing water from the culture medium aqueous solution to provide a concentrated aqueous solution containing between about 5-20 grams per liter of extracellular heteropolysaccharide, and (3) heating the concentrated aqueous solution at a temperature between about 60°-l50°C to produce an aqueous solution of heat-modified heteropolysaccharide product.

l8. A process in accordance with claim l7 wherein steps (2) and (3) are conducted as a combined procedure.

l9. A process in accordance with claim l7 wherein the growth carbon source is methanol.

20. A process in accordance with claim l7 wherein the bacterial strain is Methylophilus viscogenes ATCC 39893 or ATCC 53412 or a variant thereof, and the product is heat-modified heteropolysaccharide Poly 54.

2l. A process which comprises (l) aerobically cultivating a Methylophilus viscogenes bacterial strain in an aqueous nutrient medium containing a growth carbon source to produce a culture medium aqueous solution of an accumulated quantity of extracellular heteropolysaccharide, (2) adjusting the pH of the aqueous solution into the alkaline range to deacetylate the heteropolysaccharide, (3) removing water from the culture medium aqueous solution to provide a concentrated aqueous solution containing between about 5-20 grams per liter of extracellular deacetylated heteropolysaccharide, and (4) heating the concentrated aqueous solution at a temperature between about 60°-l50°C to produce an aqueous solution of heat-modified deacetylated heteropolysaccharide product.

22. A process in accordance with claim 2l wherein steps (3) and (4) are conducted as a combined procedure.

23. A process in accordance with claim l wherein steps (2), (3) and (4) are conducted as a combined procedure.

24. A process in accordance with claim 2l wherein the growth carbon source is methanol.

25. A process in accordance with claim 2l wherein the bacterial strain is Methylophilus viscogenes ATCC 39893 or ATCC 53412 or a variant thereof, and the product is heat-modified deacetylated heteropolysaccharide Poly 54.

0255405

# Fig.1

HEAT-MODIFIED POLY 54 VS.
POLY 54 AND XANTHAN GUM
(Biopolymers at 1 g /L )

- ■ = Heat-modified Poly 54
- + = Xanthan
- ◇ = Poly 54

0255405

# Fig.2

HEAT-MODIFIED DEACETYLATED POLY 54 vs
DEACETYLATED POLY 54 and XANTHAN GUM
(Biopolymers at 1 g / L )

- ■ = Heat-modified deacetylated Poly 54
- + = Xanthan
- ◇ = Deacetylated Poly 54

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | RESEARCH DISCLOSURE, vol. 264, April 1986, pages 182-184, Kenneth Mason Publications Ltd, New York, US; "Heteropolysaccharides" * Page 182, column 1, paragraph 2; page 182, column 2, last line * | 1-25 | C 12 P 19/04 C 08 B 37/00 |
| X | US-A-4 515 700 (D.O. HITZMAN) * Table 1; column 3, lines 19-22 * | 1 | |
| A | PATENT ABSTRACTS OF JAPAN, vol. 8, no. 266 (C-255)[1703], 6th December 1984; & JP-A-59 142 201 (KOGYO GIJUTSUIN (JAPAN)) 15-08-1984 * Abstract * | 9-16, 21-25 | |
| P,X | EP-A-0 215 623 (CELANESE CORP.) * Page 2, line 60 - page 3, line 2; claim 1 * | 9-16, 21-25 | TECHNICAL FIELDS SEARCHED (Int. Cl.4) C 12 P C 08 L |

The present search report has been drawn up for all claims

| Place of search | Date of completion of the search | Examiner |
|---|---|---|
| THE HAGUE | 06-11-1987 | SOMERVILLE F.M. |